# EUROPEAN PATENT SPECIFICATION

(11) **EP 4 373 470 B1**
(45) Date of publication and mention of the grant of the patent: **25.06.2025**
(21) Application number: 22751727.3
(22) Date of filing: 20.07.2022
(51) Int. Cl.: A61K 9/00, A61K 47/26, A61K 47/36, A61K 31/19, A61K 31/733, A61K 38/38, A23L 2/60, A23L 2/66, A23L 29/10, A23L 29/30, A23L 33/12, A23L 33/125, A23L 33/19

(54) **PHARMACEUTICAL OR FOOD SUPPLEMENT FORMULATION CONTAINING ALPHA-LACTALBUMIN AND BUTYRIC ACID OR A SALT THEREOF**
PHARMAZEUTISCHE ODER NAHRUNGSERGÄNZUNGSFORMULIERUNG MIT ALPHA-LACTALBUMIN UND BUTTERSÄURE ODER EINEM SALZ DAVON
FORMULATION PHARMACEUTIQUE OU DE COMPLÉMENT ALIMENTAIRE CONTENANT DE L'ALPHA-LACTALBUMINE ET DE L'ACIDE BUTYRIQUE OU UN SEL DE CELUI-CI

(30) Priority: 23.07.2021 IT 202100019613
(43) Date of publication of application: 29.05.2024
(73) Proprietor: Kolfarma S.r.L., 16129 Genova (IT)
(72) Inventor: MAINARDI, Paolo, 16167 Genova (IT)
(74) Representative: Botti & Ferrari S.p.A.
(86) International application number: PCT/EP2022/070337
(87) International publication number: WO 2023/001883

(56) References cited:
- WO-A1-2012/013495
- WO-A1-2019/228851
- CN-A- 106 036 140
- US-A1- 2019 216 110

## Description

### Field of application

The present invention relates to the technical field of the pharmaceutical industry or food supplement industry.

In particular, the invention relates to a pharmaceutical or food supplement formulation containing alpha-lactalbumin and butyric acid or a salt thereof with an alkaline or alkaline-earth metal.

### Prior art

Alpha-lactalbumin is known to be used as food supplement mainly because of its brain serotonergic action.

Italian Patent Application N. GE2006A000013 on behalf of the Applicant relates indeed to the use of alpha-lactalbumin in the treatment of neurological and neuropsychiatric pathologies, such as Parkinson's disease, depressive pathologies and epilepsy and European Patent N. EP 2 218 462 B1 relates to a pharmaceutical preparation comprising a SSRI or a SSNRI and alpha-lactalbumin for the treatment of depressive disorders.

Patent Application WO 2019/175274 of the present Applicant described the synergistic action of alpha-lactalbumin with short-chain fatty acids (SCFAs) in the treatment of disorders of the central nervous system, especially of those linked to serotonin deficiency, such as for example epilepsy, depression, and anxiety.

Among the SCFAs mentioned in said application, butyric acid and alkaline or alkaline-earth salts thereof are particularly preferred, and a formulation of granules of sodium butyrate in sachets is specifically exemplified, said formulation being intended to be administered in conjunction with a separate formulation of granules of alpha-lactalbumin in sachets.

The granules of sodium butyrate contain 300 mg sodium butyrate and 1500 mg hydrogenated palm oil, in addition to calcium carbonate (200 mg), maltodextrins (90 mg) and flavoring agent (10 mg).

The granules were produced by nebulizing a mixture of sodium butyrate, hydrogenated palm oil and calcium carbonate through a nozzle of a cooling chamber at about -10°C. The granules thereby obtained have a sodium butyrate-based inner core and a coating based on hydrogenated palm oil and calcium carbonate.

The coating of hydrogenated palm oil and calcium carbonate is used for masking the very unpleasant taste and smell of sodium butyrate.

In fact, the unpleasant and bitter taste of butyrate makes it problematic incorporating it in a formulation in powder form, because, when it comes into contact with the bitter taste receptors, mainly located on the tip of the tongue, it stimulates a vomiting reaction.

On the other hand, a powder formulation containing butyrate to be dissolved or dispersed in water or other liquids for oral administration would be extremely useful for administering butyrate to children and adults with swallowing problems, who have difficulties in taking coated tablets, which represent the solution adopted so far in the known art for masking the unpleasant taste and smell of butyrate.

Patent application WO/2012/013495, for example, describes pharmaceutical formulations containing an SCFA, for example butyric acid, or a salt or ester o amide thereof, a soluble or water-dispersable dietary fiber, including inulin, pectins, dextrin and maltodextrin, at least one flavoring agent and one or more excipients. The description only provides examples of formulations in the form of coated tablets of calcium butyrate, wherein the coating has the function of masking the taste and smell of butyrate and of possibly giving gastroresistance. No examples of formulations in powder form are provided.

Patent application WO 2017/160345 describes pharmaceutical preparations for oral administration of sodium phenylbutyrate, an active substance suitable for the treatment of neurodegenerative disorders such as Parkinson's disease and characterized, like butyric acid and salts thereof, by an unpleasant smell. For masking the unpleasant smell of sodium phenylbutyrate, beads are proposed that comprise a central cellulose core covered by a layer containing sodium phenylbutyrate, that is in turn covered by a smell-masking coating based on dimethylaminoethyl methacrylate, butyl methacrylate and methyl methacrylate.

Patent WO 2011/112695 describes a nutritional composition comprising whey protein powder comprising whey protein micelles and leucine, wherein the leucine content in the composition is between 20% and 40% by weight dry matter and the weight ratio of added leucine to whey protein micelles is from 1:2 to 1:3. In this composition, the bitter taste of leucine is masked.

Patent application CN 106036140 discloses a feedstuff for piglets in the form of granules, comprising nineteen ingredients, among which whey protein concentrate (30-50 parts), maltodextrin (46-60 parts), sodium butyrate (3-5 parts) and an acidulant (4-15 parts). The main ingredients of this feedstuff are, however, flour (130-200 parts) and broken rice (230-350 parts). Such feedstuff is not in powder form and it is clearly not intended for administration to a human subject, but to an animal such as a piglet that does not display any vomiting reaction in response to a bitter and unpleasant taste like that of butyrate.

Patent application WO 2019/228851 discloses a dietary supplement providing for a source of butyrate with improved organoleptic properties, such a source of butyrate being a triglyceride, in which one or two hydroxy group(s) of glycerol is/are esterified with butyric acid and the remaining one(s) is/are esterified with a long chain fatty acid having 16 to 20 carbon atoms. This dietary supplement can also comprise prebiotics, such as inulin and FOS, sweeteners and emulsifiers.

Patent application WO 2012/013495 discloses a dietary composition containing a SCFA or an ester or amide thereof, in combination with a soluble or water-dispersible dietary fibre, such as e.g. inulin or maltodextrin, and a flavoring agent.

The problem underlying the present invention was to provide a pharmaceutical or food supplement formulation in powder form for oral administration to a human subject that might allow the simultaneous administration of butyric acid or an alkaline or alkaline-earth salt thereof and alpha-lactalbumin, masking the unpleasant taste and smell of butyric acid or salts thereof, without resorting to a coated table, so that the pharmaceutical or food supplement formulation might be taken even by children and subjects having swallowing problems.

### Summary of the invention

Said problem was solved, according to the invention, by a pharmaceutical or food supplement formulation in form of a powder with a particle size lower than 1000 µm for oral administration to a human subject, comprising alpha-lactalbumin, butyric acid or a salt thereof with an alkaline or alkaline-earth metal and maltodextrins, in which the weight ratio of butyric acid or said salt thereof: alpha-lactalbumin ranges between 1:4 and 1:1.5, preferably between 1:2 and 1:1.5.

By "pharmaceutical or food supplement formulation in powder form" a formulation in the form of a powder with a particle size lower than 1000 µm, typically 10-800 µm, is meant.

Preferably, in the formulation according to the invention the weight ratio of butyric acid or said salt thereof: maltodextrins ranges between 1:3 and 1:2.

The formulation may further comprise inulin, preferably in a weight ratio of between 1:4 and 1:1, preferably between 1:2 and 1:1.3, with respect to butyric acid or said salt thereof.

The formulation may further comprise fructoligosaccharides (FOS), preferably in a weight ratio of between 1:4 and 1:1.5, preferably between 1:2.7 and 1:1.8, with respect to butyric acid or said salt thereof.

The formulation may further comprise a flavoring agent, and/or a sweetening agent, and/or an emulsifying agent.

Preferably, the flavoring agent is contained in a percentage of between 0.1% and 3%, more preferably between 0.5% and 2%, by weight of the total weight of the formulation.

Preferred flavoring agents are banana flavoring and chocolate flavoring.

Preferably, the sweetening agent is contained in a percentage of between 0.1% and 3%, more preferably between 0.5 and 2%, by weight of the total weight of the formulation. A particularly preferred sweetening agent is sucralose.

Preferably, the emulsifying agent is contained in a percentage of between 0.05% and 1%, more preferably between 0.1% and 0.5%, by weight of the total weight of the formulation. Preferably, the emulsifying agent is a non-ionic surfactant, conveniently a polysorbate, in particular polysorbate 80. The formulation according to the present invention generally comprises the ingredients that are shown, in percentages by weight of the total weight of the formulation, in the following Table 1.

**TABLE 1**

| | |
|---|---|
| Alpha-lactalbumin | 20-30 |
| Butyric acid or alkaline or alkaline-earth salt thereof | 10-18 |
| Maltodextrins | 40-50 |
| Inulin | 7-15 |
| Fructoligosaccharides | 5-10 |
| Flavoring agent | 0-3 |
| Sweetening agent | 0-3 |
| Emulsifying agent | 0-1 |

Preferably, the formulation according to the present invention contains the above-mentioned ingredients in the percentages by weight of the total weight of the formulation shown in the following Table 2.

**TABLE 2**

| | |
|---|---|
| Alpha-lactalbumin | 22-27 |
| Butyric acid or alkaline or alkaline-earth salt thereof | 12-16 |
| Maltodextrins | 42-48 |
| Inulin | 8-13 |
| Fructoligosaccharides | 6-9 |
| Flavoring agent | 0.5-2 |
| Sweetening agent | 0.5-2 |
| Emulsifying agent | 0.1-0.5 |

Preferably, the formulation according to the invention is in powder form and is intended to be dissolved in water or in an aqueous beverage, typically in a volume of about 150-200 ml.

The formulation according to present invention provides a masking of the rancid smell and taste of butyrate that is surprising, since no document of the prior art reports an effect of masking the rancid smell and taste of butyrate or of other rancid-smelling substance by means of addition of alpha-lactalbumin and maltodextrins. In fact, the above-mentioned document WO 2011/112695 reports an effect of masking a bitter and non-rancid taste by using not alpha-lactalbumin or simple whey proteins, but specific whey protein micelles obtained according to the method described in WO 2007/110411.

### DETAILED DESCRIPTION

The present invention will be further described with reference to some examples which are provided by way of non-limiting illustration.

A formulation in powder form in 4 g sachets was prepared starting from the following ingredients, as described below in Example 1 and in Example 2.

### Example 1

| | |
|---|---|
| Alpha-lactalbumin (ALAC) | 1000 mg |
| Fructoligosaccharides (FOS) | 300 mg |
| Inulin | 400 mg |
| Sodium butyrate | 625 mg |
| Banana flavoring (flavoring agent) | 37 mg |
| Maltodextrins | 1563 mg |
| Sucralose (sweetening agent) | 60 mg |
| Polysorbate 80 (emulsifying agent) | 15 mg |
| Total | 4000 mg |

The above-listed ingredients in powder form were mixed until homogeneous, and sachets for oral administration were filled with the resultant mixture.

### Example 2

| | |
|---|---|
| Alpha-lactalbumin (ALAC) | 1200 mg |
| Fructoligosaccharides (FOS) | 250 mg |
| Inulin | 400 mg |
| Sodium butyrate | 625 mg |
| Banana flavoring (flavoring agent) | 37 mg |
| Maltodextrins | 1413 mg |
| Stevia powder extract (sweetening agent) | 60 mg |
| Polysorbate 80 (emulsifying agent) | 15 mg |
| Total | 4000 mg |

The above-listed ingredients in powder form were mixed until homogeneous, and sachets for oral administration were filled with the resultant mixture.

### Example 3

| | |
|---|---|
| Alpha-lactalbumin (ALAC) | 1000 mg |
| Fructoligosaccharides (FOS) | 250 mg |
| Inulin | 320 mg |
| Sodium butyrate | 625 mg |
| Banana flavoring (flavoring agent) | 70 mg |
| Maltodextrins | 1697 mg |
| Sucralose (sweetening agent) | 30 mg |
| Polysorbate 80 (emulsifying agent) | 8 mg |
| Total | 4000 mg |

The above-listed ingredients in powder form were mixed until homogeneous, and sachets for oral administration were filled with the resultant mixture.

### Example 4 (comparative)

| | |
|---|---|
| Alpha-lactalbumin (ALAC) | 700 mg |
| Fructoligosaccharides (FOS) | 400 mg |
| Inulin | 400 mg |
| Sodium butyrate | 625 mg |
| Banana flavoring (flavoring agent) | 37 mg |
| Maltodextrins | 1763 mg |
| Sucralose (sweetening agent) | 60 mg |
| Polysorbate 80 (emulsifying agent) | 15 mg |
| Total | 4000 mg |

### Example 5 (test of organoleptic evaluation)

The solution obtained by dissolving the content of a sachet of powder according to Examples 1 and 4 in 150 ml water underwent evaluation by a panel of 12 trained tasters, who were asked to give a score from 1 to 10 (1 = minimum intensity; 10 = maximal intensity) to the organoleptic characteristics specified in the following Table 3. The scores shown in the table are the average of the scores given by each taster.

**TABLE 3**

| Characteristic | Score Example 1 | Score Example 4 |
|---|---|---|
| Rancid smell | 1.5 | 4.5 |
| Rancid taste | 1.2 | 4.1 |
| Acrid taste | 1.6 | 4.7 |
| Sweetish aftertaste | 1.8 | 4.5 |
| Bitter taste | 1.5 | 4.3 |
| Taste of fat | 1.8 | 4.7 |

In the case of the formulation according to example 1, the intensity of the negative organoleptic characteristics that are typical of butyrates was very low and all the tasters confirmed the overall palatability of the solution.

On the contrary, for the formulation according to the comparative example 4, wherein the amount of alpha-lactalbumin is lower than the amount of alpha-lactalbumin in the formulation according to the present invention, the tasters distinctly perceived the presence of negative organoleptic characteristics, in particular the rancid taste and smell that are typical of butyrates.

## Claims

1. A pharmaceutical or food supplement formulation in form of a powder with a particle size lower than 1000 µm for oral administration to a human subject, said formulation comprising alpha-lactalbumin, butyric acid or a salt thereof with an alkaline or alkaline-earth metal and maltodextrins, in which the weight ratio of butyric acid or said salt thereof : alpha-lactalbumin is between 1:4 and 1:1.5, preferably between 1:2 and 1:1.5.

2. The pharmaceutical or food supplement formulation according to claim 1, wherein the weight ratio of butyric acid or said salt thereof: maltodextrin is between 1:3 and 1:2

3. The pharmaceutical or food supplement formulation according to any one of claims 1 and 2, wherein said formulation further comprises inulin.

4. The pharmaceutical or food supplement formulation according to claim 3, wherein inulin is present in a weight ratio of between 1:4 and 1:1, preferably between 1:2 and 1:1.3, with respect to butyric acid or said salt thereof.

5. The pharmaceutical or food supplement formulation according to any one of claims 1 to 4, wherein said formulation further comprises fructoligosaccharides (FOS).

6. The pharmaceutical or food supplement formulation according to claim 5, wherein said fructoligosaccharides are present in a weight ratio of between 1: 4 and 1: 1.5, preferably between 1: 2.7 and 1 : 1.8, with respect to butyric acid or said salt thereof.

7. The pharmaceutical or food supplement formulation according to any one of claims 1 to 6, wherein said formulation further comprises a flavoring agent, preferably banana flavoring or chocolate flavoring, in a percentage of between 0.1% and 3%, preferably between 0.5% and 2%, by weight of the total weight of the formulation.

8. The pharmaceutical or food supplement formulation according to any one of claims 1 to 7, wherein said formulation further comprises a sweetening agent, preferably sucralose, in a percentage of between 0.1% and 3%, preferably between 0.5 and 2%, by weight of the total weight of the formulation.

9. The pharmaceutical or food supplement formulation according to any one of claims 1 to 8, wherein said formulation further comprises an emulsifying agent in a percentage of between 0.05% and 1%, preferably between 0.1% and 0.5%, by weight of the total weight of the formulation.

10. The pharmaceutical or food supplement formulation according to claim 9, wherein said emulsifying agent is a non-ionic surfactant, preferably polysorbate 80.

## Patentansprüche

1. Pharmazeutische oder Nahrungsergänzungsmittelformulierung in Form eines Pulvers mit einer Partikelgröße von weniger als 1000 µm zur oralen Verabreichung an einen Menschen, wobei die Formulierung alpha-Lactalbumin, Buttersäure oder ein Salz davon mit einem Alkali- oder Erdalkalimetall und Maltodextrine umfasst, wobei das Gewichtsverhältnis von Buttersäure oder dem Salz davon zu alpha-Lactalbumin zwischen 1:4 und 1:1,5, vorzugsweise zwischen 1:2 und 1:1,5, beträgt.

2. Pharmazeutische oder Nahrungsergänzungsmittelformulierung nach Anspruch 1, wobei das Gewichtsverhältnis von Buttersäure oder dem Salz davon zu Maltodextrin zwischen 1:3 und 1:2 beträgt.

3. Pharmazeutische oder Nahrungsergänzungsmittelformulierung nach einem der Ansprüche 1 und 2, wobei die Formulierung weiter Inulin umfasst.

4. Pharmazeutische oder Nahrungsergänzungsmittelformulierung nach Anspruch 3, wobei Inulin in einem Gewichtsverhältnis zwischen 1:4 und 1:1, vorzugsweise zwischen 1:2 und 1:1,3, bezogen auf die Buttersäure oder das Salz davon, vorliegt.

5. Pharmazeutische oder Nahrungsergänzungsmittelformulierung nach einem der Ansprüche 1 bis 4, wobei die Formulierung weiter Fructoligosaccharide (FOS) umfasst.

6. Pharmazeutische oder Nahrungsergänzungsmittelformulierung nach Anspruch 5, wobei die Fructoligosaccharide in einem Gewichtsverhältnis zwischen 1:4 und 1:1,5, vorzugsweise zwischen 1:2,7 und 1:1,8, bezogen auf die Buttersäure oder das Salz davon, vorliegen.

7. Pharmazeutische oder Nahrungsergänzungsmittelformulierung nach einem der Ansprüche 1 bis 6, wobei die Formulierung weiter einen Aromastoff, vorzugsweise Bananenaroma oder Schokoladenaroma, in einem Anteil zwischen 0,1 Gew.-% und 3 Gew.-%, vorzugsweise zwischen 0,5 Gew.-% und 2 Gew.-%, bezogen auf das Gesamtgewicht der Formulierung, umfasst.

8. Pharmazeutische oder Nahrungsergänzungsmittelformulierung nach einem der Ansprüche 1 bis 7, wobei die Formulierung weiter einen Süßstoff, vorzugsweise Sucralose, in einem Anteil zwischen 0,1 Gew.-% und 3 Gew.-%, vorzugsweise zwischen 0,5 und 2 Gew.-%, bezogen auf das Gesamtgewicht der Formulierung, umfasst.

9. Pharmazeutische oder Nahrungsergänzungsmittelformulierung nach einem der Ansprüche 1 bis 8, wobei die Formulierung weiter einen Emulgator in einem Anteil zwischen 0,05 Gew.-% und 1 Gew.-%, vorzugsweise zwischen 0,1 Gew.-% und 0,5 Gew.-%, bezogen auf das Gesamtgewicht der Formulierung, umfasst.

10. Pharmazeutische oder Nahrungsergänzungsmittelformulierung nach Anspruch 9, wobei der Emulgator ein nichtionisches Tensid, vorzugsweise Polysorbat 80, ist.

## Revendications

1. Formulation pharmaceutique ou de complément alimentaire sous forme de poudre, de taille de particule inférieure à 1 000 µm, destinée à une administration orale chez un sujet humain, ladite formulation comprenant de l'alpha-lactalbumine, de l'acide butyrique ou un sel de celui-ci avec un métal alcalin ou alcalino-terreux, et des maltodextrines, le rapport pondéral acide butyrique, ou ledit sel de celui-ci:alpha-lactalbumine étant compris entre 1:4 et 1:1,5, de préférence entre 1:2 et 1:1,5.

2. Formulation pharmaceutique ou de complément alimentaire selon la revendication 1, dans laquelle le rapport pondéral acide butyrique, ou ledit sel de celui-ci:maltodextrine est compris entre 1:3 et 1:2.

3. Formulation pharmaceutique ou de complément alimentaire selon l'une quelconque des revendications 1 ou 2, dans laquelle ladite formulation comprend en outre de l'inuline.

4. Formulation pharmaceutique ou de complément alimentaire selon la revendication 3, dans laquelle l'inuline est présente dans un rapport pondéral compris entre 1:4 et 1:1, de préférence entre 1:2 et 1:1,3, par rapport à l'acide butyrique ou au dit sel de celui-ci.

5. Formulation pharmaceutique ou de complément alimentaire selon l'une quelconque des revendications 1 à 4, dans laquelle ladite formulation comprend en outre des fructo-oligosaccharides (FOS).

6. Formulation pharmaceutique ou de complément alimentaire selon la revendication 5, dans laquelle lesdits fructo-oligosaccharides sont présents dans un rapport pondéral compris entre 1:4 et 1:1,5, de préférence entre 1:2,7 et 1:1,8, par rapport à l'acide butyrique ou au dit sel de celui-ci.

7. Formulation pharmaceutique ou de complément alimentaire selon l'une quelconque des revendications 1 à 6, dans laquelle ladite formulation comprend en outre un agent aromatisant, de préférence un arôme banane ou un arôme chocolat, en un pourcentage compris entre 0,1 % et 3 %, de préférence entre 0,5 % et 2 %, en poids par rapport au poids total de la formulation.

8. Formulation pharmaceutique ou de complément alimentaire selon l'une quelconque des revendications 1 à 7, dans laquelle ladite formulation comprend en outre un agent édulcorant, de préférence du sucralose, en un pourcentage compris entre 0,1 % et 3 %, de préférence entre 0,5 % et 2 %, en poids par rapport au poids total de la formulation.

9. Formulation pharmaceutique ou de complément alimentaire selon l'une quelconque des revendications 1 à 8, dans laquelle ladite formulation comprend en outre un agent émulsifiant en un pourcentage compris entre 0,05 % et 1 %, de préférence entre 0,1 % et 0,5 %, en poids par rapport au poids total de la formulation.

10. Formulation pharmaceutique ou de complément alimentaire selon la revendication 9, dans laquelle ledit agent émulsifiant est un tensioactif non ionique, de préférence du polysorbate 80.
